# EUROPEAN PATENT APPLICATION

(11) **EP 2 878 708 A1**
(43) Date of publication of application: **03.06.2015**
(21) Application number: 13005554.4
(22) Date of filing: 28.11.2013
(51) Int. Cl.: C23F 17/00, A61L 27/04, A61L 27/06, A61C 8/00, A61L 31/02, A61F 2/30, C21D 1/74, C21D 1/76, C21D 3/02, C23C 8/16, C23C 8/80, C22F 1/02, C23F 1/00

(54) **Method for the modification of the surface structure of a metal body**

(71) Applicant: Linde Aktiengesellschaft, 80331 München (DE)
(72) Inventor: Foret, Pierre, 80796 München (DE); Diamanti, Entela, 93077 Bad Abbach (DE)
(74) Representative: Gellner, Bernd

(57) **Abstract**

Method for the modification of a surface structure of a metal body, preferably for increasing the surface porosity, comprising the following steps:
- first heating of the metal body in an oxidizing atmosphere,
- cooling the metal body to room temperature, and
- pickling the surface of the metal body with at least one acid.

## Description

The invention relates to a method for the modification of the surface structure of a metal body, more preferably for increasing the surface porosity.

Stainless austenitic steels after their manufacture frequently have a smooth metal surface without structure and can therefore be frequently wetted only with difficulty. This wetting characteristic e. g. has a major influence on the adhesion and durability of paints and coatings, i.e, the application of durable coatings on such surfaces proves difficult.

In medical technology, biocompatible materials such as for example titanium are used for producting implants from these. With such implants, too, a surface that is too smooth also results in problems that are difficult to handle. Among these are for example poor contact between the implant and the human tissue in which the implant is implanted.

EP 1 935 508 A1 describes a method for the modification of the surface structure of a metal body, wherein a surface of the metal body is initially exposed to an oxidizing atmosphere and subsequently to a reducing atmosphere.

A method for microporous surface modification of implantable metallic medical articles and implantable metallic articles having such a modified surface is disclosed in US 2002/0198601 A1.

Application of such methods leads to the formation of an iron rich porous surface on the surface of a metal body, which is not corrosion resistant.

It is therefore the object of the present invention to provide a method of modifying the surface structure of a metal body, especially a stainless steel body, which at the same time offers increased corrosion resistance.

This object is achieved with a method including the features of claim 1.

The method according to the invention provides surfaces for metal bodies with good porosity and corrosion resistance characteristics.

Especially, after the first heating (i.e. heat treatment) of the metal body, the step of pickling with acids leads to a minimizing or elimination of non-corrosion resistant iron rich layers, which are the result of prior art methods. Pickling also has the effect of setting free Cr initially bound in other form such as in Cr₂O₃.

The term pickling refers to a metal surface treatment commonly used to remove impurities, such as stains, inorganic contaminents, rust or scale from ferrous metals, copper, and aluminium alloys. A solution, sometimes referred to as pickle liquor, which contains strong acids, is used to remove the surface impurities. It is commonly used to descale or clean steel in various steelmaking processes. In the context of the invention, the term pickling especially refers to use of an acid to remove the iron layer at the surface of the material.

Advantageously, the metal body comprises at least one of Cr, Ni, Mo, Ti or Nb. The presence of Cr, for example in stainless steel bodies, leads to a high corrosion resistance, as Cr reacts with air to form a protective layer of Cr oxide (CrO) on the surface of the metal body. Such layers can have thicknesses of up to 1 nm or more.

Advantageous embodiments of the method according to the invention are the subject matter of the dependent claims.

According to an especially preferred embodiment, a second heating (i.e. heat treatment) of the metal body in a reducing atmosphere, and a subsequent cooling to room temperature is effected, before the pickling is performed.

Advantageously, the first heating of the metal body in an oxidizing atmosphere is effected at temperatures between 1000°C and 1200°C, preferably 1100°C. As duration of this heat treatment, preferentially, a time span between 10 and 200 minutes, especially between 5 and 60 minutes, is selected.

The oxidizing atmosphere is preferably a wet atmosphere, especially comprising inert gas, such as N₂ or Ar, and water vapour. Such gas atmospheres are cheap to provide and provide the desired oxidizing effects. Alternatively, air or pure oxygen atmospheres can be used.

Preferably, the second heating step is conducted at temperatures of 1000°C to 1200°C, preferably 1100°C. Preferred durations are five to 120 minutes, especially 60 to 120 minutes.

Preferably, the reducing atmosphere is an H₂ atmosphere. Use of such an atmosphere leads to an effective reduction of an iron rich (Fe-rich) surface layer. Hydrogen for example, reduces iron oxide present at the surface of the metal body. A subsequent step of pickling effectively removes an iron layer.

All in all, the invention has numerous advantages compared to the prior art. With the method according to the invention, surface porosity can be customized in a simple and effective way. Depth and size of the pores can be set through suitable selection of the method parameters in the oxidation step, the reduction step and the pickling step. More preferably, stainless austenitic steels, Co-Cr-alloys, titanium and tantalum materials, which frequently have a smooth surface structure, can be prepared according to the invention so that subsequent coatings last better and are more durable.

The invention and additional details of the invention will now be explained in more detail with reference to the accompanying drawings.
Fig. 1 shows in schematically simplified form an SEM image of a metal body surface, the metal body being made of stainless steel 1.4404, after treatment with a method according to the prior art,
Fig. 2 shows, again in schematically simplified form, a corresponding SEM image after treatment with the method according to the present invention, and
Fig. 3 shows, in schematically simplified form, the effect of pickling.

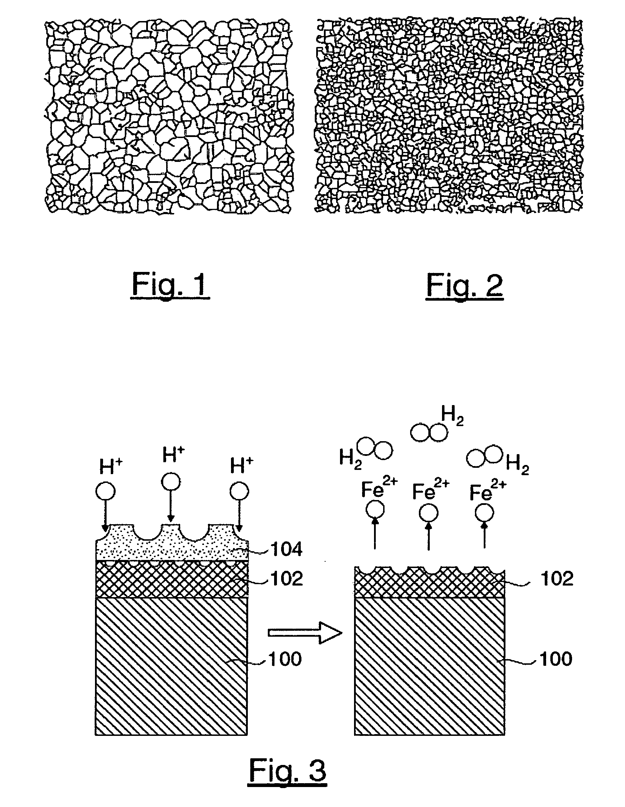

Figure 1 shows a schematically simplified SEM picture of the surface of a stainless steel after a heating step in an oxidizing atmosphere and a subsequent heating step in a reducing atmosphere. Figure 1 schematically shows a metal body after applying a method as described for example in prior art document EP 1 935 508 A1. Here, for example, a steel was oxidized in an oxygen atmosphere and subsequently reduced in a hydrogen atmosphere. Such a method produces pores with a size between 1 micrometer and 10 micrometers, wherein the pore channels that are formed reach a depth of several micrometers. In the surface of a metal body subjected to this method, the Cr level is typically low after treatment. However, a low Cr level leads to a weak corrosion resistance.

According to the present invention, it is possible to maintain a high Cr-level at the surface and enhance corrosion resistance properties of the material.

A work piece resulting from application of a preferred embodiment of the invention is shown in Figure 2.

In Figure 2, a corresponding schematically implified SEM picture after performing the method of a preferred embodiment of the invention is shown. Herein, a first heating (heat treatment) in an oxidizing atmosphere (i.e. a wet argon atmosphere) at temperatures of 1100°C, followed by cooling down to room temperature, a renewed heating (heat treatment) at 1100°C in a reducing hydrogen atmosphere, a subsequent cooling down to room temperature and a subsequent pickling with acids were performed. Pickling can be formed at room temperature, or, if desired, at any other expedient temperature. The first heating (i.e. the oxidation) is preferably performed for about 10 minutes, the second heating (i.e. reduction) is performed for preferably about 20 minutes. The pickling can be effected within a time of less than 60 minutes, especially less than 30 minutes or less than 15 minutes.

As immediately follows from the schematically shown images, porosity is substantially increased, as can be seen by the substantially smaller structures. Hereby, the total surface of the metal body is increased.

The process of pickling, which is advantageously used within the context of the present invention, is schematically shown in Fig. 3.

After a treatment in an oxidizing atmosphere and subsequently in a reducing atmosphere, a stainless steel body 100 comprising Fe and Cr will have a surface structure with lower surface layer 102 rich in Fe, Cr and O. At the same time, there will be provided an upper layer 104 which is rich only in Fe. This is due to the fact that in an oxidizing atmosphere different elements such as Fe, Cr etc. do not react with oxygen at the same speed. For example, Cr reacts faster that Fe, whereby i.a. an oxide Cr₂O₃ is formed. Layer 102 thus comprises Cr₂O₃ together with Cr in other forms, e.g. unreacted Cr. The different reaction times (i.e. different affinities to oxygen) are the reason for obtaining these different layers.

As shown in Fig. 3, a suitable acid, such as HNO₃ or HF, is applied to the iron rich layer 104 (symbolized by H⁺-ions).

The reaction of the acid with the iron in layer 104 leads to a removal of the iron rich layer 104 (symbolized by H₂ molecules and Fe²⁺ ions). Apart from the removal of layer 104, the acid applied in this pickling treatment will have the additional effect of releasing further Cr, which is initially bound within the mentioned Cr₂O₃ in layer 102. In effect, oxygen is removed from layer 102, resulting in a Cr rich composition of layer 102, to which the mentioned Cr in other forms also contributes. This Cr can then again react with oxygen present in the ambient air, to produce the desired non-corrosive layer.

As mentioned, layer 102 comprises a high Cr content. This Cr will, after removal of layer 104, react with ambient air to form a protective Cr-oxide layer on the surface of layer 102. Hereby, corrosion resistance of stainless steel body 100 is significantly enhanced. At the same time, porosity of layer 102 is sufficient for numerous applications.

Essentially, layers 102, 104 shown in Figure 3 are porous layers, created by the first heating (oxidation) and the second heating (reduction). By means of the first heating, layers are created, with essentially have the same chemical consistency as layers 102, 104, but are not provided with the desired porosity. The porosity is created by the second heating (reduction). The third step according to the present invention, i.e. the pickling step, erases the iron rich layer 104, which is, are described, not corrosion resistant. Thus, as a result of the method according to the invention, layer 102 becomes the surface layer, which is corrosion resistant, due to its content of Cr.

## Claims

1. Method for the modification of a surface structure of a metal body, preferably for increasing the surface porosity, comprising the following steps:
- first heating of the metal body in an oxidizing atmosphere,
- cooling the metal body to room temperature, and
- pickling the surface of the metal body with at least one acid.

2. Method according to claim 1, comprising a second heating of the metal body in a reducing atmosphere, following the first heating, and a further cooling to room temperature.

3. Method according to claim 1 or claim 2, wherein the metal body is made of steel, especially stainless or austenitic steel, Co-Cr-alloys, titanium or tantalum materials.

4. Method according to any one of the preceding claims wherein first heating is effected at temperatures of up to 1000°C, 1100°C, 1200°C or 1300°C, preferably at temperatures between1000°C and 1200°C.

5. Method according to any one of the preceding claims, wherein the first heating occurs in a wet atmosphere, especially comprising an inert gas and water vapour.

6. Method according to any one of the preceding claims 2 to 4, wherein the second heating is effected at temperatures of up to to 1000°C, 1100°C, 1200°C or 1300°C, especially at temperatures between 1000°C and 1200°C.

7. Method according to any one of the preceding claims, wherein the second heating is effected in an hydrogen atmosphere.

8. Method according to any one of the preceding claims, wherein the step of pickling is effected with at least one of the following acids: NHO₃, HF.
